(19) Europäisches Patentamt
European Patent Office
Office européen des brevets

(11) **EP 2 520 240 B1**

(12) **EUROPÄISCHE PATENTSCHRIFT**

(45) Veröffentlichungstag und Bekanntmachung des Hinweises auf die Patenterteilung:
**11.01.2017   Patentblatt 2017/02**

(51) Int Cl.:
***A61B 18/12*** *(2006.01)*

(21) Anmeldenummer: **11164639.4**

(22) Anmeldetag: **03.05.2011**

(54) **Einrichtung zur Gewerbefusion oder Koagulation durch elektrische Einwirkung mit negativer Quellimpedanz**

Device for tissue fusion or coagulation by means of electric force with negative source impedance

Dispositif de fusion ou de coagulation tissulaire par un effet électrique à impédance de source négative

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**07.11.2012   Patentblatt 2012/45**

(73) Patentinhaber: **ERBE Elektromedizin GmbH
72072 Tübingen (DE)**

(72) Erfinder:
• **Schall, Heiko
72622 Nürtingen (DE)**

• **Brodbeck, Achim
72555 Metzingen (DE)**
• **Fritz, Martin
72072 Tübingen (DE)**

(74) Vertreter: **Rüger, Barthelt & Abel
Patentanwälte
Webergasse 3
73728 Esslingen (DE)**

(56) Entgegenhaltungen:
WO-A1-99/65406          WO-A2-2010/142438
DE-A1-102008 038 314    US-A1- 2007 173 803
US-A1- 2009 234 351     US-A1- 2010 179 534

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Einrichtung zur Gewebefusion oder Koagulation mittels mindestens einer Elektrode, die einen Strom in das zu behandelnde Gewebe einleitet.

Aus der WO 2010/142438 A2 ist ein HF-chirurgisches Gerät bekannt, das zur Speisung eines chirurgischen Instruments mit HF-Energie dient. Das Gerät und das Instrument sind untereinander durch ein Kabel verbunden. Das Gerät weist eine Wirkstrom-Berecheneinheit auf, um den an das Instrument gelieferten Strom zu erfassen und die gelieferte Spannung anhand des Stroms zu regulieren. Dabei können beliebige positive Innenwiderstände und alternativ auch ein negativer Innenwiderstand erzeugt werden, der den positiven Innenwiderstand des Kabels kompensiert. Auf diese Weise kann zwischen den Elektroden ein Innenwiderstand von Null eingestellt werden, ohne dass dazu die Spannung an den E-lektroden abgegriffen werden müsste.

Die Gewebefusion oder Koagulation durch eine Elektrode, die lokal Strom in Gewebe und/oder Gewebeflüssigkeit einleitet, ist z.B. aus der EP 1 862 137 A1 bekannt. Während der Behandlung entstehen dabei in der Umgebung der Elektrode im Gewebe Effekte, die zu einer Änderung der Gewebeimpedanz führen. Zu Beginn der Einwirkung weist das Gewebe eine Anfangsimpedanz auf, die schon kurz nach Beginn der Stromeinleitung auf einen geringeren Wert absinkt, was als "Phase I" bezeichnet wird. Nach einiger Zeit steigt die Gewebeimpedanz wieder an, was als "Phase II" bezeichnet wird. Die Gewebeimpedanz erreicht in der Phase II normalerweise Werte, die deutlich über der Anfangsimpedanz liegen. Der Impedanzanstieg flacht sich dann ab und erreicht gegebenenfalls einen stabilen Endwert, was als "Phase III" bezeichnet wird.

Die zeitlichen Längen der Phasen I und II und die Steilheit des Impedanzabfalls und des Impedanzanstiegs bestimmen die Qualität des erreichten chirurgischen Ergebnisses.

Das System gemäß der EP 1 862 137 A1 versucht deshalb, den zeitlichen Verlauf der Gewebeimpedanz mit einer Soll-Kurve in Übereinstimmung zu bringen. Zu diesem Zweck vergleicht das System den auf eine geeignete Weise gemessenen Ist-Wert der Gewebeimpedanz fortwährend mit dem für den jeweiligen Zeitpunkt geltenden Soll-Wert. Wird eine Abweichung festgestellt, wird eine Gegenmaßnahme, wie beispielsweise eine Vergrößerung oder Verringerung der Energieeinleitung in das Gewebe vorgenommen. Der hier zugrunde liegende regelungstechnische Ansatz kann jedoch an Grenzen stoßen, wenn Regelabweichungen auftreten, die zu einer irreversiblen Änderung der Gewebestruktur geführt haben, wie z.B. eine vorzeitige Eiweißdenaturierung.

Es wird deshalb nach einer robusten und verlässlichen Einrichtung zur Durchführung der Gewebefusion oder Koagulation insbesondere der dauerhaften Gefäßversiegelung gesucht.

Diese Aufgabe wird mit der Einrichtung nach Anspruch 1 gelöst:

Die Einrichtung beruht auf der Einleitung eines Stroms in ein biologisches Gewebe mittels einer Elektrode, sowie der Ausleitung des Stroms mittels einer Gegenelektrode. Zwischen beiden Elektroden ist ein Strompfad durch das biologische Gewebe ausgebildet, das einen elektrischen Gewebewiderstand darstellt. Im Verlauf der Stromeinleitung ergeben sich verschiedene Phasen I, II sowie gegebenenfalls III des zeitlichen Verlaufs des Gewebewiderstands. Die zur Speisung der Elektrode verwendete elektrische Quelle ist vorzugsweise eine gesteuerte (HF-)Spannungsquelle. Die Ausgangsspannung der Quelle wird prozessentsprechend geführt. Als Führungsgröße wird in zumindest einer der Phasen, vorzugsweise in Phase II, der zwischen der Elektrode und der Gegenelektrode fließende elektrische Strom genutzt. Vorzugsweise wird die Größe des gemessenen Stroms zumindest in der Phase II so zur Regulierung der Ausgangsspannung der Quelle herangezogen, dass sich ein Gesamtverhalten der Quelle ergibt, das einer Quelle mit einem gewünschten, vorzugsweise negativen Innenwiderstand entspricht. Der Strom wird vorzugsweise fortwährend, d.h. kontinuierlich oder auch in diskreten Zeitpunkten, z.B. periodisch gemessen.

Eine Änderung des Innenwiderstands der Quelle kann schaltungstechnisch simuliert werden, indem eine Quelle mit festem Innenwiderstand jedoch variabler, steuerbarer Spannung genutzt wird. Die Spannung wird anhand des gemessenen, durch das Gewebe fließenden Stroms so geführt, dass von der Elektrode und Gegenelektrode aus gesehen ein gewünschter Innenwiderstand der Quelle gegeben ist. Es ist insbesondere möglich, negative Innenwiderstände einzustellen. Dies vorzugsweise während der Phase II. Der Betrag des (vorzugsweise negativen) Innenwiderstands ist dabei vorzugsweise kleiner als der Gewebewiderstand. Dies stellt eine stabile Arbeitsweise sicher.

Der Begriff Gewebewiderstand bezeichnet wahlweise den Realteil oder den Betrag der komplexen Gewebeimpedanz.

Zur Steuerung der Gewebeimpedanz während der Phase II können vorzugsweise als Vorgabe für die Quelle ein rampenförmiger Spannungsanstieg kombiniert mit einem den Widerstand steuernden Term genutzt werden. Vorzugsweise wird dazu ein funktionaler Zusammenhang definiert, der durch

$$u_a = m \cdot t + u_0 - \frac{m \cdot R_G}{AR_0}$$

festgelegt ist. In dieser Gleichung wird ein rampenförmiger Spannungsanstieg durch die Parameter $m$ und $u_0$ festgelegt. Der letzte Term in dieser Gleichung stellt einen Widerstandsterm dar, in dem der Gewebewiderstand im Verhältnis zu einer Bezugswiderstandsände-

rung $AR_0$, als zeitliche Ableitung eines Bezugswiderstands eingeht. Dieser Term steuert die Ausgangsspannung derart, dass bei Vergrößerung des Gewebewiderstands $R_G$ die Spannung $u_a$ verringert wird. Bei Durchführung des Verfahrens ergibt sich der zeitliche Verlauf dieses Gewebewiderstands durch entsprechende Einstellung der an der Elektrode anliegenden Spannung $u_a$. Die Bildung der an der Elektrode anliegenden Spannung $u_a$ unter Ausnutzung eines negativen Innenwiderstands liefert die gleiche Wirkung, wie die Steuerung der Gewebeimpedanz mit nachfolgenden Beziehungen. Es gilt dabei $u_a = u + R_i * i_a$. $u = m*t + u_0$. $i_a$ ist der durch die Elektrode fließende Strom. Der (simulierte) Innenwiderstand $R_i$ ist vorzugsweise negativ. Hier steuert der Term $-R_i * i_a$ die Ausgangsspannung derart, dass bei Vergrößerung des Gewebewiderstands $R_G$ die Spannung $u_a$ verringert wird.

Wenn in Phase II mit einem negativen Innenwiderstand und einer geeigneten Spannungsrampe gearbeitet wird, kann unabhängig von den variierenden biologischen Gegebenheiten in der Umgebung der Elektrode erreicht werden, dass die für die Phase II erforderliche Zeit T2 konstant wird. Mit anderen Worten, die Phase II kann in festgelegter Prozesszeit ausgeführt werden. Dies dient der Prozesssicherheit. Es kann sichergestellt werden, dass die Ablaufsteuerung zum Durchführen der Phase II mit einem Timer zu einem gewünschten chirurgischen Ergebnis gleichbleibender Qualität führt. Insbesondere wird durch den rampenförmigen Spannungsanstieg, verbunden mit einem negativen Innenwiderstand der Quelle während der Phase II, vermieden, dass durch vorschnellen, zu starken Energieeintrag in das Gewebe Effekte erzielt werden, die sich durch späteres Reduzieren des Energieeintrags nicht mehr rückgängig machen lassen. Die vorgeschlagene Regel oder Steuerstrategie ist deshalb für die Regulierung des teilweise irreversibel ablaufenden und somit hochgradig nichtlinearen Prozesses in der Phase II besonders zweckmäßig.

Üblicherweise umfasst eine Einrichtung zur Durchführung des Verfahrens mindestens ein Gerät zur Bereitstellung elektrischer Leistung, wobei dieses Gerät eine elektrische Quelle umfasst. Weiter gehört zu der Einrichtung ein Instrument mit mindestens einer Elektrode, das an das Gerät angeschlossen ist. Die Elektrode dient zur Einleitung des elektrischen Stroms in das Gewebe. Ein Steuermodul erfasst den Strom und steuert die Quelle entsprechend. Während in Phase I z.B. mit einem festen Strom, einer festen Leistung, einer festen Spannung und/oder einem festen vorzugsweise positiven Innenwiderstand der Quelle gearbeitet wird, wird in Phase II vorzugsweise mit einem rampenförmigen Spannungsanstieg und einem vorzugsweise negativen Innenwiderstand der Quelle gearbeitet.

Weiter vorzugsweise enthält eine solche Einrichtung eine Instrumentenerkennung. Mittels der Instrumentenerkennung können einzelne Parameter, mit denen das Verfahren in Phase II arbeitet, festgelegt werden. Solche Parameter sind beispielsweise der Spannungsanstieg m der Rampe, eine Grund- oder Bezugsspannung $u_0$ und oder der gewünschte, vorzugsweise negative Innenwiderstand $R_i$. Zu der Instrumentenerkennung können Codestecker oder sonstige Speichermittel, wie beispielsweise ein an dem Instrument vorgesehener Speicher oder jegliche andere geeignete Mittel vorgesehen sein, die es ermöglichen, wenigstens einen der oben genannten Parameter dem Instrument entsprechend auszuwählen. Auf diese Weise können für verschiedene Instrumente jeweils gleiche Prozesszeiten T2 erreicht werden. Damit wird eine hohe Qualität der Gewebefusion der Gewebekoagulation, dauerhaften Gefäßversiegelung sowie auch der Gefäßanastomose erreicht. Auch erhöht die feste Dauer der Phase II die Behandlungssicherheit, weil der Chirurg sich an eine einheitliche Einwirkungszeit des Instruments auf das Gewebe gewöhnt und einstellt.

Bei den im Vorstehenden genannten Instrumenten kann es sich um monopolare, vorzugsweise aber um bipolare Instrumente, wie beispielsweise Gefäßklemmen handeln, deren beide Klemmschenkel als Elektrode und Gegenelektrode ausgebildet sind. Solche Gefäßklemmen dienen der dauerhaften Gefäßversiegelung. Ein solches Gerät klemmt ein Gefäß ab und verschließt dieses durch Verkleben gegenüberliegender aufeinander gepresster Gewebewände. Außerdem kann ein solches Instrument ein Messer zum Durchtrennen des verschlossenen Gefäßes enthalten.

Monopolare, im Rahmen der Erfindung benutzte Instrumente können beispielsweise platten- oder kugelförmige, schleifenförmige oder anderweitig ausgebildete Elektroden aufweisen. Die Gegenelektrode nimmt hier an dem chirurgischen Effekt nicht teil. Sie wird beispielsweise als Neutralelektrode an dem Patienten befestigt.

Weitere Einzelheiten vorteilhafter Ausführungsformen der Erfindung ergeben sich aus Unteransprüchen, der Zeichnung oder der Beschreibung. Es zeigen:

Fig. 1 eine schematische Darstellung einer Einrichtung zur Gefäßversiegelung,

Fig. 2 ein Blockschaltbild der Einrichtung nach Fig. 1 zur Veranschaulichung der Funktionsweise,

Fig. 3 ein Diagramm zur Veranschaulichung des Verlaufs des Gewebewiderstands über der Zeit und

Fig. 4 ein Gefäß während einer Gefäßversiegelung in schematischer, ausschnittsweiser Längsschnittdarstellung.

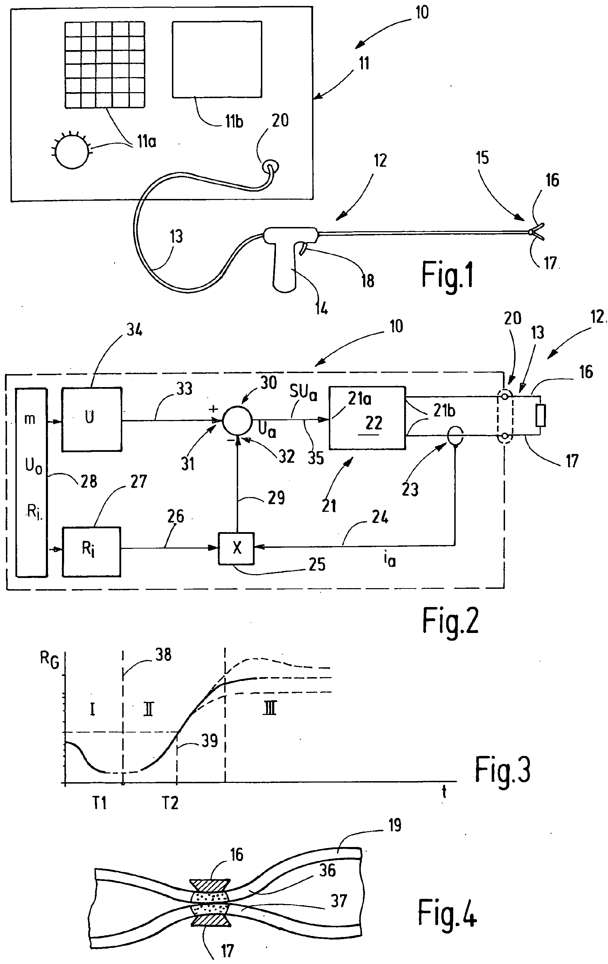

In Fig. 1 ist eine Einrichtung 10 veranschaulicht, die hier als Beispiel für unterschiedliche, zur Gewebekoagulation geeignete Einrichtungen steht. Die Einrichtung 10 umfasst ein Gerät 11 zur Speisung und zum Betrieb eines chirurgischen Instruments 12. Das Gerät 11 weist ein oder mehrere Bedienelemente 11a und mindestens ein Anzeigeelement 11b in Form einer Anzeigeeinrichtung, z.B. ein Display auf. Das Instrument 12 ist über eine Leitung 13 mit dem Gerät 11 verbunden. Das Instrument 12 wird über die Leitung 13 von dem Gerät 11 mit Spannung versorgt.

Das Instrument 12 ist im vorliegenden Ausführungsbeispiel eine bipolare Gefäßklemme mit einem Handgriff 14 und einem Werkzeug 15. Letzteres umfasst eine Elektrode 16 und eine Gegenelektrode 17, von denen wenigstens eine, im vorliegenden Ausführungsbeispiel beide beweglich gelagert sind. Sie können durch Betätigung eines Handhebels 18 aufeinander zu und voneinander weg bewegt werden. Bei dem Instrument 12 muss es sich nicht zwingend um ein bipolares Instrument handeln, wie es hier dargestellt ist. Es können auch monopolare Instrumente zum Einsatz kommen, die nur eine Elektrode aufweisen. Die Gegenelektrode ist dann beispielsweise eine am Patienten möglichst großflächig zu befestigende Neutralelektrode.

Die elektrische Grundstruktur der Einrichtung 10 ist in Fig. 2 als Blockschaltbild veranschaulicht. Ein Widerstand $R_G$ symbolisiert hier die Impedanz, im einfachsten Fall den Ohmschen Widerstand des zwischen den Elektroden 16, 17 gefassten Gewebes 19. Solches Gewebe 19 kann beispielsweise ein Blutgefäß sein, wie es in Fig. 4 ausschnittsweise und schematisch angedeutet ist.

Die Elektroden 16, 17 sind in Fig. 2 lediglich auf ihre elektrische Funktion reduziert, als Leitungen dargestellt. Sie sind über die Leitung 13 und einen Steckverbinder 20 an das Gerät 11 angeschlossen.

Zur Versorgung des Instruments 12 mit elektrischer Leistung dient eine Quelle 21, die durch eine Gleich- oder Wechselspannungsquelle, vorzugsweise durch einen HF-Generator 22 gebildet wird. Dieser stellt an seinem Ausgang 21b hochfrequente Wechselspannung, z.B. im Bereich mehrerer hundert Kilohertz und bedarfsweise mehreren hundert Volt, bei schneidenden, kontaktlosen Koagulationen oder abtragenden Anwendungen auch über tausend Volt bereit. Die Amplitude der HF-Spannung $u_a$ entspricht einem an seinem Steuereingang 21a empfangenen Spannungssignal s$u_a$. Der HF-Generator 22 liefert die HF-Leistung an die Elektrode 16 und die Gegenelektrode 17. In einer der entsprechenden Leitungen, z.B. in der zu der Gegenelektrode führenden Leitung, kann ein Stromsensor 23 angeordnet sein, der ein Signal erzeugt, das den durch das Gewebe fließenden Strom kennzeichnet. Vorzugsweise kennzeichnet das Signal oder den Effektivwert den Betrag des Stroms und ist somit selbst kein Hochfrequenzsignal. Das Signal kann über einen vorzugsweise elektronischen Gleichrichter bereitgestellt werden, wobei dieser wiederum vorzugsweise ein geglättetes Signal liefert. Die Glättung kann mit einem RC-Glied durchgeführt werden. Die Zeitkonstante ist vorzugsweise kleiner als eine Sekunde, um schnelle Stromanstiege oder Stromabfälle in dem Signal adäquat abzubilden.

Das den Strom kennzeichnende Signal wird über einen Signalpfad 24 als Eingangssignal zu einem Multiplizierer 25 geleitet. Der Multiplizierer 25 erhält über einen weiteren Signalpfad 26 ein weiteres Eingangssignal $R_i$, das von einem Block 27 bereitgestellt wird. Das Signal $R_i$ kennzeichnet einen Innenwiderstand, der als Generatorinnenwiderstand zwischen der Elektrode 16 und der Gegenelektrode 17 wirksam werden soll. Das Signal $R_i$ kann positive oder negative Werte annehmen. Seine Größe und sein Vorzeichen werden von einer Systemsteuerung 28 bestimmt, die den Block 27 entsprechend steuert.

Der Multiplizierer 25 multipliziert die über die Signalpfade 24, 26 erhaltenen Signale und liefert das Produkt über einen weiteren Signalpfad 29 an einen Summierer 30. Der Summierer 30 weist einen nichtinvertierenden Eingang 31 und einen invertierenden Eingang 32 auf und bildet somit die vorzeichenbehaftete Summe, also die Differenz zwischen den Signalen, die er an den beiden Eingängen 31, 32 erhält. Der Summierer 30 kann durch einen Differenzverstärker gebildet werden.

Während der Signalpfad 29 an den invertierenden Eingang 32 angeschlossen ist, erhält der nichtinvertierende Eingang 31 über einen Signalpfad 33 ein Spannungssignal u aus einem Block 34. Das Spannungssignal u kann einen vorgegebenen Zeitverlauf aufweisen, der von dem Block 34 gebildet wird. Das Signal u folgt vorzugsweise einem Zeitverlauf entsprechend:

$$u = m*t + u_0.$$

Dies ist eine Geradengleichung. Der Anstieg m und der konstante Term $u_0$ werden von der Systemsteuerung 28 zweckentsprechend vorgegeben.

Von dem Summierer 30 führt ein Signalpfad 35 zu dem Steuereingang 21a des HF-Generators 22. Das dort vorhandene Steuersignal $u_a$ genügt der Beziehung:

$$u_a = u - R_i * i_a$$

wobei $i_a$ ein Signal ist, das den mit dem Stromsensor 23 erfassten durch das Gewebe fließenden Strom kennzeichnet.

Der Multiplizierer 25, der Summierer 30, die Blöcke 27, 34, die Systemsteuerung 28 und der Gleichrichter bzw. Effektivwertbildner des Stromsignals können durch spezifische Schaltungen oder durch Software realisiert werden. Sie können insbesondere Programme oder Programmabschnitte eines oder mehrerer Mikrocontroller sein.

Die insoweit beschriebene Einrichtung 10 arbeitet wie folgt:

Es wird davon ausgegangen, dass ein Operateur mit dem Instrument 12 ein Blutgefäß 19 verschließen möchte. Er fasst das Blutgefäß 19 deshalb zwischen der Elektrode 16 und der Gegenelektrode 17 und betätigt den Handhebel 18, um gegenüberliegende Abschnitte 36, 37 der Wand des Blutgefäßes 19 aneinander zu drücken. Eine geeignete Maßnahme, beispielsweise die Betätigung des Handhebels 18 oder auch die Betätigung eines weiteren Schalters, der z.B. an dem Handgriff 14 angebracht sein kann, oder eines Fußschalters, aktiviert dann die Leistungsabgabe des Geräts 11. Solange das Blutgefäß 19 noch nicht bestromt war, weist es eine Anfangsimpedanz von z.B. 50 Ohm oder einen ähnlichen Wert auf. In Fig. 3 ist dies in dem Zeitintervall T1 links dargestellt. Auf der senkrechten Skala ist der Gewebestand $R_G$ logarithmisch veranschaulicht.

Mit der Bestromung des Gewebes nimmt der Gewebewiderstand $R_G$ relativ schnell ab. Z.B. öffnen sich einzelne Zellen und es bilden sich elektrolytgefüllte Strompfade. In dieser ersten Phase I kann nach einem geeigneten, fest vorgegebenen Schema gearbeitet werden, beispielsweise mit konstanter Spannung $u_0$, mit konstanter Leistung, mit konstantem Strom oder nach anderweitigen Kriterien. Dies kann fest vorgegeben sein. Es ist auch möglich, das Gerät 11 so zu gestalten, dass die einzelnen Modi für die Phase I wählbar oder einstellbar sind. Es ist auch möglich, eine Instrumentenerkennung vorzusehen, die beispielsweise mit einem in dem Instrument 12 vorgesehenen Speicher zusammenwirkt und dementsprechend die Betriebsart des HF-Generators 22 bzw. der Quelle 21 einstellt.

Während der Phase I wird der Fortgang des Prozesses überwacht, um den Beginn der Phase II erkennen zu können. Die Überwachung kann beispielsweise durch eine Überwachung des fließenden Stroms oder durch Überwachung anderer physikalischer Größen, wie insbesondere auch des Phasenwinkels einer elektrischen Größe, des Stroms, der Spannung zwischen der Elektrode 16 und der Gegenelektrode 17 oder des Gewebewiderstands $R_G$ erfolgen. Der Zeitverlauf desselben in Fig. 3 zeigt, dass es möglich ist, das erste Wiederansteigen des Gewebewiderstands $R_G$ nach Durchlaufen eines Minimums als Zeichen für den Beginn der Phase II zu nehmen. Dies ist in Fig. 3 durch eine vertikale gestrichelte Linie 38 veranschaulicht. Es ist jedoch auch möglich, andere Grenzwerte oder Schwellwerte zu setzen. Beispielsweise kann der Beginn der Phase II auch als derjenige Zeitpunkt definiert sein, bei dem erstmalig wieder ein Gewebewiderstand $R_G$ erreicht wird, der deutlich über dem zu Beginn der Phase I gemessenen Gewebewiderstand liegt. Dies ist in Fig. 3 durch eine zweite vertikale gestrichelte Linie 39 veranschaulicht.

Unabhängig davon, wie der Anfangszeitpunkt der Phase II definiert ist, geht das Gerät 11 zu Beginn der Phase II in eine Phase-II-Betriebsart über, die sich von der zuvor eingenommenen Betriebsart unterscheidet. In der Phase II simuliert das Gerät 11 einen negativen Innenwiderstand $R_i$ der Quelle 21, im vorliegenden Ausführungsbeispiel des HF-Generators 22. Dieser hat physikalisch einen geringen positiven Innenwiderstand oder einen Innenwiderstand von praktisch Null. Jedoch wird die Ausgangsspannung $u_a$ dem gemessenen Strom $i_a$ so nachgeführt, dass sich aus Sicht des Gewebewiderstands $R_G$ ein negativer Innenwiderstand $R_i$ der speisenden Quelle 21 ergibt. Vorzugsweise gilt dabei, dass der Betrag des negativen Innenwiderstands $R_i$ geringer ist als der Gewebewiderstand $R_G$.

In dem Gerät 11 wird das den Strom $i_a$ kennzeichnende Signal mit dem vorzugsweise negativen Innenwiderstand $R_i$ multipliziert und das entstehende Signal auf dem Signalpfad 29 zu dem Summierer 30 geliefert. Hier wird die Differenz zwischen einer Spannung u und dem Produkt aus $i_a$ und $R_i$ gebildet. Die Spannung u folgt einer zeitlichen Rampe mit dem Anfangswert $u_0$ und dem Anstieg m. Die Größen $R_i$, m und $u_0$ sind vorzugsweise über eine Instrumentenerkennung instrumentenspezifisch festgelegt. Vorzugsweise können sie vom Bediener nicht oder jedenfalls nicht ohne weiteres geändert werden.

Aufgrund der Nutzung eines negativen Innenwiderstand $R_i$ folgt dass ein abnehmender, durch das Gewebe fließender Strom $i_a$ zu einer abnehmenden Generatorspannung führt und umgekehrt. Auf diese Weise gilt, dass ein zunehmender Gewebewiderstand $R_G$ zu einer abnehmenden Spannung $u_a$ führt. Durch die zusätzliche Wirkung der Rampenfunktion gilt, dass je schneller der Gewebewiderstand $R_G$ steigt, desto langsamer steigen die Spannung $u_a$ und somit der Energieeintrag in das Gewebe. Dies führt in der Phase II zu einer festgelegten Prozesszeit T2, siehe Fig. 3. Die Prozesszeit T2 kann somit für größere oder kleinere Gefäße und unabhängig von physiologischen Unterschieden zwischen einzelnen Patienten weitgehend konstant festgelegt werden und führt somit zu einer hohen Behandlungssicherheit und verlässlich hohen Qualität der Gewebefusion.

Das Ende der Phase II ist erreicht, wenn die festgelegte Prozesszeit T2 abgelaufen ist. Der Koagulationsprozess kann dann in der Phase III noch nach geeigneten Vorgaben fortgesetzt oder auch beendet werden.

Bei dem zur Gewebefusion oder auch zur Koagulation geeigneten Verfahren wird nach Behandlungsbeginn des Gewebes, d.h. nach Durchlaufen einer Phase I, eine Phase II begonnen, innerhalb derer das biologische Gewebe für eine bestimmte festgelegte Prozesszeit mit moderatem Energieeintrag behandelt wird. Durch Festlegung eines negativen Innenwiderstands einer speisenden Quelle 21 kann erreicht werden, dass der Prozess in der Phase II eine gleich bleibende Behandlungszeit in Anspruch nimmt und somit eine vorzeitige Gewebeaustrocknung vermieden wird. Es wird eine ausreichende und verlässliche Verklebung der beteiligten Eiweiße im feuchten Milieu erreicht.

Bezugszeichenliste:

| | |
|---|---|
| 10 | Einrichtung |
| 11 | Gerät |
| 11a | Bedienelemente |
| 11b | Anzeigeelement |
| 12 | Instrument |
| 13 | Leitung |
| 14 | Handgriff |
| 15 | Werkzeug |
| 16 | Elektrode |
| 17 | Gegenelektrode |
| 18 | Handhebel |
| 19 | biologisches Gewebe/Blutgefäß |
| 20 | Steckverbinder |
| 21 | Quelle |
| 21a | Steuereingang |
| 21b | Ausgang |
| 22 | HF-Generator |
| 23 | Stromsensor |
| 24 | Signalpfad |
| 25 | Multiplizierer, Steuermodul |
| 26 | Signalpfad |
| 27 | Block |
| 28 | Systemsteuerung |
| 29 | Signalpfad |
| 30 | Summierer, Steuermodul |
| 31 | nichtinvertierender Eingang |
| 32 | invertierender Eingang |
| 33 | Signalpfad |
| 34 | Block |
| 35 | Signalpfad |
| 36, 37 | Abschnitte der Gefäßwand |
| 38, 39 | gestrichelte Linien - Beginn von Phase II |
| $AR_0$ | Bezugswiderstandsänderung |
| $u_a$ | Ausgangsspannung, Steuersignal |
| $u_0$ | konstanter Spannungsterm |
| m | Spannungsanstieg |
| u | Spannungssignal |
| $R_i$ | Signal zur Kennzeichnung eines Innenwiderstands |
| $R_G$ | Gewebewiderstand |
| $su_a$ | Spannungssignal |
| $i_a$ | Strom |

**Patentansprüche**

1. Einrichtung (10) zur Gewebefusion oder Koagulation mittels hochfrequenten Wechselstroms, mit einer elektrischen Quelle (21), die einen Ausgang (21b) aufweist, an dem sie ausgeführt ist, mit einem zumindest zeitweilig negativen Innenwiderstand eine hochfrequente Wechselspannung zu liefern, mit mindestens einer Elektrode (16), die an den Ausgang (21b) angeschlossen und zur Bewirkung eines chirurgischen Effekts mit einem biologischen Gewebe (19) in Wechselwirkung zu bringen ist, mit mindestens einer Gegenelektrode (17), die an den Ausgang (21b) angeschlossen und mit dem biologischen Gewebe (19) in elektrische Verbindung zu bringen ist **dadurch gekennzeichnet**, die Einrichtung ausgeführt ist, die Spannung anhand eines gemessenen, durch das Gewebe (21) fließenden Stroms so zu führten, dass von der Elektrode (16) und Gegenelektrode (17) aus gesehen zumindest während einer Phase der Gewebefusion bzw. Koagluation ein gewünschter negativer Innenwiderstand (Ri) der Quelle (21) gegeben ist.

2. Einrichtung nach Anspruch 1, **dadurch gekennzeichnet, dass** sie:

   einen Stromsensor (23) zur fortwährenden Bestimmung des zwischen der Elektrode (16) und der Gegenelektrode (17) fließenden elektrischen Stroms aufweist, wobei der Stromsensor (23) ausgeführt ist, ein den elektrischen Strom kennzeichnendes Signal zu erzeugen, und ferner ein Steuermodul (25, 30) aufweist, das mit dem Steuereingang (21a) der Quelle (21) verbunden ist und ausgeführt ist, dieser anhand des von dem Stromsensor (23) abgegebenen Signals ein Steuersignal ($u_a$) bereitzustellen, das an einem Steuereingang (21a) der Quelle (21) liegt und die Größe von der elektrischen Quelle (21) abgegeben Spannung festlegt,

   wobei das Steuermodul (25, 30) ausgeführt ist, in wenigstens einer von mehreren Betriebsphasen den negativen Innenwiderstand (Ri) der elektrischen Quelle festzulegen.

3. Einrichtung nach Anspruch 1, **dadurch gekennzeichnet, dass** sie ausgeführt ist, den Innenwiderstand ($R_i$) während einer ersten Phase (I), in der die Gewebeimpedanz ($R_G$) zunächst eine sinkende und dann etwa gleichbleibende Tendenz hat, auf einen positiven Wert festzulegen.

4. Einrichtung nach Anspruch 1, **dadurch gekennzeichnet, dass** sie ausgeführt ist, den Innenwiderstand ($R_i$) während einer zweiten Phase (II), in der die Gewebeimpedanz ($R_G$) eine ansteigende oder zunächst eine gleichbleibende und dann ansteigende Tendenz hat, auf den negativen Wert festzulegen.

5. Einrichtung nach Anspruch 1, **dadurch gekennzeichnet, dass** zur Steuerung der Spannung in Abhängigkeit von dem Strom ($i_a$) ein funktionaler Zusammenhang festgelegt ist.

6. Einrichtung nach Anspruch 5, **dadurch gekennzeichnet, dass** mit dem funktionalen Zusammen-

hang ein variabler Innenwiderstand ($R_i$) der elektrischen Quelle (21) simuliert wird.

7. Einrichtung nach Anspruch 1, **dadurch gekennzeichnet, dass** sie dazu eingerichtet ist, zur Steuerung der von der Quelle (21) abgegebenen Spannung $u_a$ einen funktionaler Zusammenhang gemäß:

$$u_a = m \cdot t + u_0 - \frac{m \cdot R_G}{AR_0}$$

zu nutzen, in dem m einen Spannungsanstieg, t die Zeit, $u_0$ eine Spannungskonstante, $AR_0$ eine Bezugswiderstandsänderung, $R_G$ einen Gewebewiderstand charakterisieren.

8. Einrichtung nach Anspruch 1, **dadurch gekennzeichnet, dass** die Steuerung der Quelle (21) in zumindest zwei Phasen (I, II) unterteilt ist, deren erste durch eine Gewebewiderstandsminderung und deren zweite durch einen Gewebewiderstandsanstieg ($R_G$) gekennzeichnet ist, wobei zumindest die zweite Phase (II) eine festgelegte konstante Dauer (T2) aufweist.

9. Einrichtung nach Anspruch 8, **dadurch gekennzeichnet, dass** die Einrichtung derart ausgelegt ist, dass der Übergang von der ersten Phase (I) zu der zweiten Phase (II) anhand des Durchlaufens eines Widerstandsminimums des Gewebewiderstands ($R_G$) erkannt wird.

**Claims**

1. Device (10) for tissue fusion or coagulation by means of high-frequency alternating current,
with an electrical source (21) having an output (21b) at which it is configured to supply a high-frequency AC voltage, for at least part of the time with a negative internal resistance,
with at least one electrode (16) which is connected to the output (21b) and to achieve a surgical effect, is to be brought into interaction with a biological tissue (19),
with at least one counter electrode (17) which is connected to the output (21b) and is to be brought into electrical connection with the biological tissue (19),
**characterised in that** the device is configured to guide the voltage by means of a measured current flowing through the tissue ($21^1$),
that viewed from the electrode (16) and counter electrode (17), at least during a phase of tissue fusion or coagulation, a desired negative internal resistance (Ri) of the source exists.

2. Device according to claim 1, **characterised in that**
it comprises a current sensor (23) for continuous determination of an electrical current flowing between the electrode (16) and counter electrode (17), wherein the current sensor (23) is configured to produce a signal characteristic of the electrical current, and furthermore
comprises a control module (25, 30) connected to the control input (21 a) of the source (21) and configured to provide this with a control signal ($u_a$) from the signal output by the current sensor (23), which signal lies at a control input (21 a) of the source (21) and establishes the value of the voltage output by the electrical source (21),
wherein the control module (25, 30) is configured to establish the negative internal resistance ($R_i$) of the electrical source in at least one of several operating phases.

3. Device according to claim 1, **characterised in that** it is configured to establish the internal resistance ($R_i$) at a positive value during a first phase (I) in which the tissue impedance (RG) has first a falling and then an approximately constant tendency.

4. Device according to claim 1, **characterised in that** it is configured to establish the internal resistance ($R_i$) at the negative value during a second phase (II) in which the tissue impedance ($R_G$) has a rising or first a constant and then a rising tendency.

5. Device according to claim 1, **characterised in that** a functional correlation is established to control the voltage as a function of the current ($i_a$).

6. Device according to claim 5, **characterised in that** a variable internal resistance ($R_i$) of the electrical source (21) is simulated using the functional correlation.

7. Device according to claim 1, **characterised in that** it is configured, for controlling the voltage $u_a$ emitted by the source (21), to use a functional correlation according to:

$$u_a = m \cdot t + u_0 - \frac{m \cdot R_G}{AR_0}$$

wherein m characterises a voltage rise, t the time, $u_0$ a voltage constant, $AR_0$ a reference resistance change, $R_G$ a tissue resistance.

8. Device according to claim 1, **characterised in that** the controlling of the source (21) is divided into at least two phases (I, II), the first of which is **characterised by** a tissue resistance reduction and the second by a tissue resistance increase ($R_G$), wherein at least the second phase (II) has an established constant duration (T2).

9. Device according to claim 8, **characterised in that** the device is configured such that the transition from the first phase (I) to the second phase (II) is detected from the passage through a resistance minimum of the tissue resistance ($R_G$).


**Revendications**

1. Dispositif (10) de fusion tissulaire ou de coagulation au moyen de courant alternatif à haute fréquence, comportant une source électrique (21) qui présente une sortie (21 b), laquelle est configurée de manière à délivrer à ladite sortie, au moyen d'une résistance intérieure, au moins temporairement négative, une tension alternative à haute fréquence, comportant au moins une électrode (16) qui est raccordée à la sortie (21b) et qui doit être mise sous effet alternatif en vue de provoquer un effet chirurgical sur un tissu biologique (19), comportant une contre-électrode (17) qui est raccordée à la sortie (21b) et qui doit être mise en liaison électrique avec le tissu biologique (19), **caractérisé en ce que** le dispositif est configuré de manière à gouverner la tension à l'aide d'un courant mesuré qui s'écoule dans le tissu (21) de telle manière que, vu à partir de l'électrode (16) et de la contre-électrode (17), au moins au cours d'une phase de la fusion du tissu ou de la coagulation, une résistance intérieure ($R_i$) négative souhaitée à la source (21) soit conférée.

2. Dispositif selon la revendication 1 **caractérisé en ce qu'** il présente un capteur de courant (23) destiné à la détermination en continu du courant électrique qui s'écoule entre l'électrode (16) et la contre-électrode (17), le capteur de courant (23) étant configuré en vue de produire un signal définissant le courant électrique et **en ce qu'**il présente en outre un module de commande (25, 30) qui est relié à l'entrée de commande (21a) de la source (21) et qui est configuré de manière à fournir à celle-ci, en fonction du signal délivré par le capteur de courant (23), un signal de commande ($u_a$), qui se situe à une entrée de commande (21a) de la source (21) et qui fixe la valeur de la tension délivrée par la source électrique (21), le module de commande (25, 30) étant configuré de manière à fixer, au moins dans l'une des plusieurs phases de fonctionnement, la résistance intérieure négative ($R_i$) de la source électrique.

3. Dispositif selon la revendication 1 **caractérisé en ce qu'** il est configuré de manière à, au cours d'une première phase (I) pendant laquelle l'impédance ($R_G$) du tissu a d'abord une tendance à baisser puis à rester approximativement égale, fixer la résistance interne ($R_i$) à une valeur positive.

4. Dispositif selon la revendication 1, **caractérisé en ce qu'** il est configuré de manière à, au cours d'une deuxième phase (II), dans laquelle l'impédance du tissu ($R_G$) a une tendance à augmenter ou a d'abord une tendance à rester égale, puis a une tendance à augmenter, fixer la résistance interne ($R_i$) à la valeur négative.

5. Dispositif selon la revendication 1 **caractérisé en ce qu'**, en vue de la commande de la tension en fonction du courant ($i_a$), une liaison fonctionnelle est fixée.

6. Dispositif selon la revendication 5, **caractérisé en ce que** grâce à la liaison fonctionnelle une résistance intérieure variable ($R_i$) de la source électrique (21) est simulée.

7. Dispositif selon la revendication 1 **caractérisé en ce qu'**il est agencé, en vue de la commande de la tension ($u_a$) délivrée par la source (21), de manière à utiliser une liaison fonctionnelle, selon :

$$u_a = m \cdot t + u0 - \frac{m \cdot R_G}{AR_0}$$

dans laquelle m caractérise une augmentation de la tension, t le temps, $u_0$ une constante de tension, $AR_0$ une variation de la résistance de référence, $R_G$ une résistance tissulaire.

8. Dispositif selon la revendication 1 **caractérisé en ce que** la commande de la source (21) est subdivisée en au moins deux phases (I, II) dont la première est **caractérisée par** une réduction de la résistance tissulaire et dont la deuxième est **caractérisée par** une augmentation de la résistance tissulaire ($R_G$), au moins la deuxième phase (II) présentant une durée constante (T2) qui est fixée.

9. Dispositif selon la revendication 8 **caractérisé en ce que** le dispositif est agencé de telle façon que la transition entre la première phase (I) et la deuxième phase (II) est reconnue grâce au transit dans un minimum de résistance de la résistance tissulaire ($R_G$).

Fig.1

Fig.2

Fig.3

Fig.4

**EP 2 520 240 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010142438 A2
- EP 1862137 A1